(19) Europäisches Patentamt
European Patent Office
Office européen des brevets

(11) Veröffentlichungsnummer: **0 118 862**
**B1**

(12) # EUROPÄISCHE PATENTSCHRIFT

(45) Veröffentlichungstag der Patentschrift: **14.08.85**

(51) Int. Cl.⁴: **C 07 C 79/46,** C 07 C 76/06

(21) Anmeldenummer: **84102335.1**

(22) Anmeldetag: **05.03.84**

(54) **Verfahren zur Isolierung von m-Nitrobenzoesäuremethylester.**

(30) Priorität: **10.03.83 DE 3308460**

(43) Veröffentlichungstag der Anmeldung: **19.09.84 Patentblatt 84/38**

(45) Bekanntmachung des Hinweises auf die Patenterteilung: **14.08.85 Patentblatt 85/33**

(84) Benannte Vertragsstaaten: **CH DE FR GB LI**

(56) Entgegenhaltungen:
**DE - A - 2 540 900**
**GB - A - 2 022 584**

(73) Patentinhaber: **BASF Aktiengesellschaft, Carl-Bosch-Strasse 38, D-6700 Ludwigshafen (DE)**

(72) Erfinder: **Hackenberger, Alfred, Dr., Luitpoldstrasse 77, D-6700 LUdwigshafen (DE)**
Erfinder: **Patsch, Manfred, Dr., Fritz-Wendel-Strasse 4, D-6706 Wachenheim (DE)**
Erfinder: **Gaeng, Manfred, Dr., An der Tuchbleiche 11, D-6712 Bobenheim-Roxheim (DE)**

**EP 0 118 862 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Isolierung von m-Nitrobenzoesäuremethylester durch Behandlung eines Isomerengemischs mit 10 bis 1000 Gew.-% Wasser in Gegenwart von 0,05 bis 20 Gew.-% Emulgator, bezogen auf die Gewichtsmenge Isomerengemisch,
a) in einer ersten Stufe bei einer Temperatur von 55 bis 100° C und einem pH-Wert von 2 bis 8 und dann
b) in einer zweiten Stufe bei einer Temperatur von 10 bis 40° C und einem pH-Wert von mindestens 9.

Es ist bekannt (Houben-Weyl, Methoden der organischen Chemie, Bd. 10/1, S. 620), dass bei der Nitrierung von Benzoesäureestern ein Gemisch der drei möglichen Isomeren entsteht. Anteile von 72,6% (Nitrierung mit Salpetersäure bei 25° C) bzw. 81 bis 85% (Einwirkung von Nitriersäure auf in $H_2SO_4$ gelöstem Ester bei 5 bis 15° C) m-Nitrobenzoesäuremethylester im Gemisch werden genannt. Durch die letztgenannte Arbeitsweise (Org. Synth., Coll. Vol. I, 372-374 [N.Y. 1948]) gelingt es, ein Isomerengemisch zu erhalten, das einen hohen Anteil an dem gewünschten m-Isomeren enthält. Nach der Umsetzung wird das Gemisch auf Eis gegossen und abgesaugt. Das Filtergut wird mit Wasser gewaschen und mit eiskaltem Methanol behandelt, um den Anteil an o-Ester und andere Verunreinigungen zu entfernen. Dann wird das Gemisch erneut abgesaugt, das Filtergut mit kaltem Methanol gewaschen und getrocknet.

Bei den beschriebenen Verfahren ist es jedoch nicht möglich, reinen m-Nitroester zu erhalten. Beim Eintragen des Nitriergemisches in Wasser fällt ein Gemisch der drei isomeren Nitrobenzoesäuremethylester an, das etwa 10% o-Isomeres und bis 2% p-Isomeres enthält.

Ausserdem enthält das Isomerengemisch geringe Mengen an Dinitrobenzoesäuremethylester, Nitrophenolverbindungen und weitere Nebenprodukte unbekannter Natur, die in wechselnden Mengen vorhanden sein können. Zur Reinigung der gewünschten 3-Nitroverbindung muss deshalb die vorgenannte zusätzliche Umkristallisation aus einem organischen Lösungsmittel wie etwa Methanol vorgenommen werden. Dadurch ergeben sich erhebliche Verluste an Wertprodukt, zusätzliche Lösungsmittelkosten und die beim Arbeiten mit Lösungsmitteln im grosstechnischen Betrieb zusätzlich notwendigen Massnahmen im Hinblick auf Betriebssicherheit, Rückgewinnung des Lösungsmittels, Gesundheits- und Umweltschutz.

Es wurde nun gefunden, dass man m-Nitrobenzoesäuremethylester aus dem Gemisch von m-, p- und o-Nitrobenzoesäuremethylester vorteilhaft erhält, wenn man das Isomerengemisch mit Wasser in einer Menge von 10 bis 1000 Gew.-% in Gegenwart von 0,05 bis 20 Gew.-% Emulgator, bezogen auf die Gewichtsmenge Isomerengemisch,
a) in einer ersten Stufe bei einer Temperatur von 55 bis 100° C und einem pH-Wert von 2 bis 8 und dann
b) in einer zweiten Stufe bei einer Temperatur von 10 bis 40° C und einem pH-Wert von mindestens 9 behandelt.

Die Nitrierung kann durch die folgenden Formeln wiedergegeben werden:

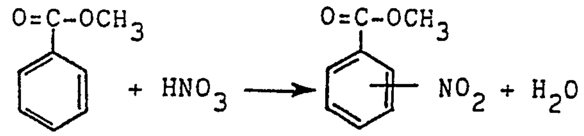

Im Vergleich zu den bekannten Verfahren liefert das Verfahren nach der Erfindung auf einfacherem und wirtschaftlicherem Weg m-Nitrobenzoesäuremethylester in besser Ausbeute und Reinheit. Organische Lösungsmittel werden nicht verwendet. Das erfindungsgemässe Verfahren ist mit Bezug auf Sicherheit im Betrieb und Schutz des Betriebspersonals vergleichsweise besser und umweltfreundlicher. Alle diese vorteilhaften Ergebnisse sind im Hinblick auf den Stand der Technik überraschend.

Die Nitrierung von Benzoesäuremethylester mit Salpetersäure zur Herstellung der m-Nitroverbindung kann nach den bekannten Verfahren, z. B. einem der in vorgenannten Veröffentlichungen beschriebenen Verfahren, durchgeführt werden. Man nitriert zweckmässig mit Salpetersäure, vorteilhaft konzentrierter oder rauchender Salpetersäure, in Gegenwart von Schwefelsäure, vorteilhaft hochkonzentrierter oder rauchender Schwefelsäure. Ggf. kann man ganz oder teilweise Salpetersäure und Schwefelsäure durch Nitriersäure, ein Gemisch der beiden Säuren, ersetzen. Im allgemeinen verwendet man eine 85 bis 100 gew.-%ige Salpetersäure und eine 98 bis 100 gew.-%ige Schwefelsäure. Zweckmässig wählt man in dem Gemisch von Salpetersäure und Schwefelsäure, der Nitriersäure, ein Verhältnis von 0,2 bis 2 mol Salpetersäure je Mol Schwefelsäure. In der Regel verwendet man von 2 bis 10 mol, vorzugsweise von 2 bis 3 mol Salpetersäure je Mol Ester. Anstelle von Salpetersäure können auch diese Säure im Reaktionsgemisch bildende Stoffe, z. B. anorganische Nitrate wie Natrium- oder Kaliumnitrat, in entsprechenden Mengen zur Anwendung gelangen. Ggf. verwendet man Harnstoff als Nitrierkatalysator, zweckmässig in einer Menge von 10 bis 100, vorzugsweise von 45 bis 55 Gew.-%, bezogen auf den Methylester. Die Umsetzung wird im allgemeinen bei einer Temperatur zwischen 0 und +40° C, vorzugsweise zwischen 10 und 30° C, drucklos oder unter Druck, diskontinuierlich oder kontinuierlich durchgeführt. Lösungsmedium ist die Säure bzw. das Säuregemisch im allgemeinen selbst, ggf. im Gemisch mit Wasser als entsprechend konzentriertes Säuregemisch.

Die Reaktion kann wie folgt durchgeführt werden: Ein Gemisch von Ausgangsstoff und Schwefelsäure wird auf die Reaktionstemperatur gebracht und dann unter Rühren langsam, z. B. während 15 bis 30 min, mit Salpetersäure, ggf. im Gemisch mit Schwefelsäure, versetzt. Das Gemisch wird noch 1 bis 3 h bei der Reaktionstemperatur gehalten.

Als Ausgangsgemisch des erfindungsgemässen Verfahrens kann man den nach einer der bekannten Aufarbeitungen anfallenden m-Nitrobenzoesäuremethylester verwenden. Vorzugsweise wird man aber eines der vorgenannten Nitrierverfahren durchführen und nach der Reaktion das gebildete Isomerengemisch des Nitrobenzoesäuremethylesters aus dem Reaktionsgemisch durch Verdünnen mit Wasser bei einer Temperatur von 85 bis 100° C, vorzugsweise von 95 bis 100° C, ausfällen und abtrennen. Die Fällung kann drucklos oder unter Druck, kontinuierlich oder diskontinuierlich durchgeführt werden. Im allgemeinen verwendet man 100 bis 500, vorzugsweise 100 bis 200 Gew.-% Wasser, bezogen auf die Gesamtmenge an verwendeter Säure (berechnet 100%). Zweckmässig gibt man das Reaktionsgemisch nach Ende der Reaktion direkt und mit solcher Geschwindigkeit in das vorgelegte Verdünnungswasser, dass vorgenannte Fällungstemperatur eingestellt und während der Vermischung eingehalten wird. Bei vorgenanntem Verhältnis Wasser zu Säure beträgt die Vermischungszeit zweckmässig 5 bis 15 min. Anschliessend wird das verdünnte Gemisch vorteilhaft noch 10 bis 20 min bei der Fällungstemperatur gerührt und dann abfiltriert. Das Filtergut kann nun direkt bzw. zweckmässig nach Waschen mit Wasser der erfindungsgemässen Behandlung zugeführt werden. Durch die Fällung wird das erhaltene Isomerengemisch nicht in deutlichem Mass zersetzt. Es kann als feuchtes Filtergut ohne weiteres Trocknen der Reinigung unterworfen werden.

Man erhält so je nach Nitrierung und Art der Aufarbeitung ein Gemisch von 70 bis 94 Gew.-% m-, 0,25 bis 20 Gew.-% o- und 0,25 bis 10 Gew.-% p-Ester, das noch mit 0,1 bis 2 Gew.-% Dinitrobenzoesäuremethylester, 0,1 bis 1,5 Gew.-% Nitrophenolverbindung und 0,3 bis 2 Gew.-% weiterer Verunreinigungen verunreinigt ist. Als Isomerengemisch wird hier das Gemisch der 3 Isomere ohne die vorgenannten Verunreinigungen definiert.

Die Wasserbehandlung (Reinigung) wird in der Stufe a bei einer Temperatur von 55 bis 100° C, vorteilhaft von 60 bis 85° C, in der Stufe b von 10 bis 40° C, vorteilhaft 20 bis 30° C, drucklos oder unter Druck, kontinuierlich oder diskontinuierlich durchgeführt. In der Regel liegt die Temperatur der Stufe a über dem Schmelzpunkt des m-Nitrobenzoesäuremethylesters. Man verwendet 10 bis 1000, vorteilhaft 50 bis 200 Gew.-% Wasser und/oder 0,05 bis 20, vorteilhaft 0,5 bis 5 Gew.-% Emulgator, bezogen auf die Gewichtsmenge Isomerengemisch. Zweckmässig beträgt Behandlungszeit in Stufe a 0,1 bis 10, vorteilhaft 0,2 bis 2 h, in Stufe b 0,5 bis 25, vorteilhaft 2 bis 5 h. Man stellt in Stufe a pH-Werte von 2 bis 8, vorteilhaft 5 bis 7, in Stufe b pH-Werte von mindestens 9, zweckmässig von 9 bis 14, vorteilhaft 10 bis 14, insbesondere 12 bis 13,5, ein.

Als Emulgatoren bezeichnet man bei dem erfindungsgemässen Verfahren einen Stoff oder ein Stoffgemisch, der oder das die Bildung einer Emulsion des o-Nitrobenzoesäuremethylesters in Wasser ermöglicht bzw. erleichtert. Dazu geeignet sind Stoffe, wie sie z. B. im Kapitel Emulgatoren in Ullmanns Enzyklopädie der technischen Chemie (Bd. 10, S. 455-464, 4. Aufl. [1975]) beschrieben sind. Zweckmässig sind nichtionische Emulgatoren wie Fettsäureester, Fettamine, Fettsäureamide und Polyglykolether auf der Basis von Polyethylenoxid oder Polypropylenoxid, bevorzugt des Typs N 301 bis N 318 und N 401 bis N 405 (Ullmann, loc. cit., S. 456), insbesondere des Typs N 303 und N 304, z. B. von Fettsäuren mit 5 bis 26 Kohlenstoffatomen und ein- oder mehrwertigen Alkoholen von 1 bis 5 Kohlenstoffatomen, kondensiert mit 20 bis 60 mol Ethylenoxid oder Propylenoxid; anionische Salze, zweckmässig Alkalisalze, vorteilhaft Natrium- und Kaliumsalze, von Sulfonaten, zweckmässig des Typs A 201 bis A 212 (Ullmann, loc. cit., S. 457), vorteilhaft des Typs A 206, insbesondere Estern der Sulfobernsteinsäure mit Alkoholen von 1 bis 12 C-Atomen; organische Phosphorsäureverbindungen, vorteilhaft des Typs A 401 bis A 407 (Ullmann, loc. cit., S. 458), bevorzugt des Typs A 401, insbesondere Ester der Phosphorsäure mit einem Alkanol mit 1 bis 20 Kohlenstoffatomen bzw. deren Polyglykolethern mit 20 bis 70 Kohlenstoffatomen; kationische Emulgatoren, zweckmässig des Typs K 101 bis K 111 (Ullmann, loc. cit., S. 458), vorteilhaft des Typs K 103 und K 104, bevorzugt solche mit Chlorid als Anion, insbesondere mit quaternären Ammoniumkationen mit 4 Alkylgruppen von 1 bis 20 C-Atomen oder 3 Alkylgruppen von 1 bis 20 C-Atomen und einer Benzylgruppe als Substituenten.

Die Reinigung kann wie folgt durchgeführt werden:

Benzoesäuremethylester wird entsprechend einer der vorgenannten Arbeitsweisen nitriert, das Reaktionsgemisch dann mit Wasser verdünnt und filtriert. Das noch feuchte Filtergut enthält zweckmässig 2 bis 35 Gew.-% Wasser. Es wird nun ohne Trocknung mit Wasser und einer erfindungsgemässen Menge Emulgator versetzt, wobei die vorgenannte Gesamtmenge Wasser eingestellt wird. Man kann auch zuerst den Emulgator und dann das Wasser dem Reaktionsgemisch zusetzen. Ebenfalls kann man den Emulgator schon vor, während und nach der Nitrierung dem Ausgangsgemisch bzw. Reaktionsgemisch der Nitrierung zusetzen. Es bildet sich eine Suspension, die zuerst bei der höheren Temperatur der Stufe a und dann bei einer tieferen Temperatur der Stufe b unter den vorgenannten Bedingungen (Reaktionszeit, Reaktionstemperatur, Reaktions-pH) der beiden Stufen gehalten wird. Dann wird der m-Nitrobenzoesäuremethylester in üblicher Weise, z. B. durch Filtration, Wäsche des Filterguts mit Wasser, erneute Filtration und Trocknung, abgetrennt. Man erhält im allgemeinen einen m-Nitrobenzoesäuremethylester einer Reinheit von 98,9 bis 99,8 Gew.-%, wobei ein restlicher Anteil von ca. 0,05 Gew.-% p-Nitrobenzoesäuremethylester, ca. 0,1 bis 1 Gew.-% o-Nitrobenzoesäuremethylester

und ca. 0,05 Gew.-% weitere Verunreinigungen verbleiben können. Am Ende der Stufe b bildet der Hauptteil der o-Isomere mit dem Emulgator eine Emulsion und bleibt nach Abtrennung des m-Nitrobenzoesäuremethylesters im Filtrat. Aus dem Filtrat kann die o-Isomere durch Wasserdampfdestillation abgetrennt werden. Der Hauptteil der p-Isomere wird in Stufe b verseift und die so gebildete p-Nitrobenzoesäure wird z. B. bei einem sauren pH-Wert ausgefällt und durch Filtration abgetrennt. Die abgetrennten Nebenprodukte o-Nitrobenzoesäuremethylester und p- Nitrobenzoesäure können für weitere Synthesen verwendet werden.

Der nach dem Verfahren der Erfindung erhältliche m-Nitrobenzoesäuremethylester ist ein wertvoller Ausgangsstoff für die Herstellung von Farbstoffen und Pflanzenschutzmitteln. Da ein praktisch reiner m-Nitrobenzoesäuremethylester erhalten wird, der nur noch Spuren an Verunreinigungen enthält, ist dieser Endstoff somit für die direkte Weiterverarbeitung wie etwa die katalytische reduktive Alkylierung zum m-N,N-Dimethylaminobenzoesäuremethylester vorteilhaft. Aus dem m-N,N-Dimethylaminobenzoesäuremethylester kann man die m-N,N-Dimethylaminobenzoesäure herstellen, die ein Zwischenprodukt für die Herstellung des Farbstoffs Kristallviolettlacton ist. Bezüglich der Verwendung wird ausserdem auf die vorgenannten Veröffentlichungen verwiesen.

*Beispiel 1:*

1000 g wasserfeuchter m-Nitrobenzoesäuremethylester (enthaltend 810 g rohen Nitroester der Zusammensetzung: o-Isomere: 8,8 Gew.-%; p-Isomere: 9 Gew.-%; m-Isomere: 77,3 Gew.-%; Dinitroverbindung und sonstige Verunreinigungen: 4,9 Gew.-%) wurden in 1000 g Wasser suspendiert und mit 25 g eines Emulgators, der durch Kondensation von Rizinusöl mit 48 mol Ethylenoxid hergestellt wurde, versetzt. Die Suspension wurde auf 75° C erwärmt und mit NaOH auf pH 6,3 gestellt. Danach wurde das Gemisch 30 min bei 75° C gerührt und auf 22° C abgekühlt. Man gab 17 g NaOH zu, wobei ein pH von 13 erreicht wurde. Die Suspension wurde 2,5 h bei 25° C gerührt. Danach wurde das Gemisch abgesaugt, das Filtergut mit Wasser gewaschen und abgesaugt. Es wurden nach Trocknung 590 g m-Nitrobenzoesäuremethylester (Fp. 78° C) von 99,6%iger Reinheit erhalten. Dies entspricht einer Ausbeute von 93,8% (bezogen auf eingesetzten m-Nitrobenzoesäureester).

*Beispiele 2 bis 5:*

Je 1000 g wasserfeuchter 3-Nitrobenzoesäuremethylester (enthaltend 950 g roher Nitroester der Zusammensetzung: o-Isomere: 10,2 Gew.-%; p-Isomere: 5,2 Gew.-%; m- Isomere: 83,4%; sonstige Verunreinigungen: 1,2 Gew.-%) wurden entsprechend der in Beispiel 1 beschriebenen Verfahrensweise gereinigt. Menge und chemische Bezeichnung des jeweils eingesetzten Emulgators sowie die erzielten Ausbeuten und Reinheiten sind in der folgenden Tabelle zusammengefasst.

*Tabelle*

| Beispiel | eingesetzter Emulgator | Menge an Emulgator (in g) | Ausbeute an m-Nitrobenzoesäuremethylester (%) | Reinheit (%) |
|---|---|---|---|---|
| 2 | wie Beispiel 1 | 25 | 95,3 | 99,8 |
| 3 | Natriumsalz des Sulfobernsteinsäuredi-(2-ethyl-hexyl)-esters | 50 | 96,7 | 99,1 |
| 4 | Dimethylbenzylalkyl-($C_{12}$-$C_{14}$)-ammoniumchlorid | 25 | 98,1 | 99,3 |
| 5 | saurer Ester der Phosphorsäure und eines mit 12 mol Ethylenoxid und 6 mol Propylenoxid kondensierten $C_{13}$-$C_{15}$-Alkanols | 25 | 99,2 | 99,4 |

## Patentanspruch

Verfahren zur Isolierung von m-Nitrobenzoesäuremethylester aus dem Gemisch von m-, p- und o-Nitrobenzoesäuremethylester, dadurch gekennzeichnet, dass man das Isomerengemisch mit Wasser in einer Menge von 10 bis 1000 Gew.-% in Gegenwart von 0,05 bis 20 Gew.-% Emulgator, bezogen auf die Gewichtsmenge Isomerengemisch,

a) in einer 1. Stufe bei einer Temperatur von 55 bis 100° C und einem pH-Wert von 2 bis 8 und dann

b) in einer 2. Stufe bei einer Temperatur von 10 bis 40° C und einem pH-Wert von mindestens 9 behandelt.

## Claim

A process for isolating methyl m-nitrobenzoate from a mixture of methyl m-, p- and o-nitrobenzoates, wherein the isomer mixture is treated with from 10 to 1000% by weight of water in the presence of from 0.05 to 20% by weight of an

emulsifier, the percentages being based on the weight of the isomer mixture,
(*a*) in a first stage at from 55 to 100° C and a pH from 2 to 8, and then
(*b*) in a second stage at from 10 to 40° C and a pH of at least 9.

## Revendication

Procédé pour l'isolement d'ester méthylique d'acide m-nitrobenzoïque à partir du mélange d'esters méthyliques d'acides m-, p- et o-nitrobenzoïques, caractérisé en ce qu'on traite le mélange d'isomères par l'eau dans une proportion de 10 à 1000% en poids en présence de 0,05 à 20% en poids d'un émulsionnant, par rapport à la quantité en poids du mélange d'isomères,
a) en un premier temps à une température de 55 à 100° C et à un pH de 2 à 8, puis
b) en un second temps à une température de 10 à 40° C et à un pH d'au moins 9.